# EUROPEAN PATENT APPLICATION

(11) **EP 4 465 306 A1**
(43) Date of publication of application: **20.11.2024**
(21) Application number: 23305775.1
(22) Date of filing: 16.05.2023
(51) Int. Cl.: G16H 50/20, G16H 50/30, G16H 10/00, A61B 5/346, A61B 5/361, A61B 5/363

(54) **METHOD AND SYSTEM FOR PREDICTING A RISK OF VENTRICULAR ARRHYTHMIA**

(71) Applicant: Koninklijke Philips N.V., 5656 AG Eindhoven (NL)
(72) Inventor: CARBONATI, Tanner, 5656 Eindhoven (NL); FIORINA, Laurent, 5656 Eindhoven (NL)
(74) Representative: Philips Intellectual Property & Standards

(57) **Abstract**

Proposed concepts thus aim to provide schemes, solutions, concept, designs, methods and systems pertaining to predicting a risk of ventricular arrhythmia in a subject. In particular, embodiments aim to provide a method for predicting a risk of ventricular arrhythmia in a subject by utilizing a machine-learning model to process an input cardiovascular signal. The machine-learning model is trained to predict a ventricular arrhythmia risk score indicating a risk of future ventricular arrhythmia occurrence. The risk score can then be used to determine a risk value describing a predicted risk of future ventricular arrhythmia occurrence for the subject.

## Description

### FIELD OF THE INVENTION

This invention relates to the field of ventricular arrhythmia, and in particular, to the field of predicting a risk of ventricular arrhythmia.

### BACKGROUND OF THE INVENTION

Sudden cardiac death (SCD) accounts for 5 million deaths each year caused predominantly by ventricular arrhythmias (VA). Long-term predictors of SCD have been studied using left ventricular ejection fraction (LVEF), however, these have reported low sensitivity and specificity. Additionally, no near-term predictors have been developed or validated. An accurate near-term prediction of ventricular arrhythmias could enable actional prevention of SCD.

### SUMMARY OF THE INVENTION

The invention is defined by the claims.

According to examples in accordance with an aspect of the invention, there is provided a method for predicting a risk of ventricular arrhythmia in a subject.

The method comprises: obtaining an input cardiovascular signal of the subject; providing the input cardiovascular signal as an input to a machine-learning model, the machine-learning model being trained to predict, based on the input cardiovascular signal, a ventricular arrhythmia risk score indicating a risk of future ventricular arrhythmia occurrence; and determining a risk value describing a predicted risk of future ventricular arrhythmia occurrence for the subject based on the ventricular arrhythmia score.

Proposed concepts thus aim to provide schemes, solutions, concept, designs, methods and systems pertaining to predicting a risk of ventricular arrhythmia in a subject. In particular, embodiments aim to provide a method for predicting a risk of ventricular arrhythmia in a subject by utilizing a machine-learning model to process an input cardiovascular signal. The machine-learning model is trained to predict a ventricular arrhythmia risk score indicating a risk of future ventricular arrhythmia occurrence. The risk score can then be used to determine a risk value describing a predicted risk of future ventricular arrhythmia occurrence for the subject.

In other words, it is proposed that by obtaining a cardiovascular signal of a subject, e.g. an ECG or PPG signal, a risk value describing a predicted risk of future ventricular arrhythmia occurrence for the subject can ultimately be determined. This can be done via the provision of the cardiovascular signal to a machine-learning model trained for this purpose. The predicted risk of future ventricular arrhythmia occurrence can be understood as the risk of ventricular arrhythmia occurring in the subject in the near-future, e.g. in the next month or two weeks or week or day, etc.

By processing a cardiovascular signal with a machine-learning model, which may comprise multiple machine-learning algorithms, a ventricular arrhythmia risk score can be predicted. Other information can also be input into the machine-learning model to make more reliable and/or accurate predictions. Based on the output ventricular arrhythmia risk score, a risk value can then be determined. The risk value may simply be the same as the risk score, or the risk score may be adjusted in some way to determine the risk value, e.g., accounting for other factors, or converting the score into, for example, a percentage-likelihood of ventricular arrhythmia occurring in the near-future.

In a simple example, an input ECG signal of the subject may be obtained and provided to multiple machine-learning algorithms which can each generate a respective ventricular arrhythmia risk prediction. The ventricular arrhythmia risk predictions can then be combined in some way to generate a risk value. For instance, the risk value may be a simple average of the risk predictions, or a weighted average, or the risk predictions may be fed into an algorithm which is trained to output a risk value for a plurality of risk predictions. By having multiple, separate machine-learning algorithms, different relevant factors may be considered and processed in different ways that are more effective and efficient than if all factors were to be considered by one large machine-learning algorithm.

This invention may be of particular use in clinical settings where ECG and/or PPG monitoring equipment is already present or is already being used. Information from these sensors is then able to be leveraged to determine a predicted risk of future ventricular arrythmia occurrence for the subject in an efficient, effective, and accurate manner without the need of any further equipment or data. A near-time prediction of ventricular arrythmia from any of these devices would therefore allow actionable prevention through the use of: medication; follow-up monitoring; and PVC or VT ablation to avoid future life-threatening arrhythmia.

Ultimately, an improved method for predicting a risk of ventricular arrhythmia in a subject may be provided.

In some embodiments, the machine-learning model may comprise a plurality of machine-learning algorithms, wherein each machine-learning algorithm generates a respective ventricular arrhythmia risk prediction, and wherein the ventricular arrhythmia risk score may comprise the plurality of ventricular arrhythmia risk predictions. The use of different algorithms allows for more flexibility in the method with different factors being considered and processed differently. This can facilitate the provision of more efficient and/or reliable risk predictions that can ultimately result in a more efficient, effective and/or reliable risk score.

In some embodiments, determining the risk value based on the ventricular arrhythmia score may comprise averaging the plurality of ventricular arrhythmia risk predictions. The plurality of risk predictions can be averaged to produce a single risk value. In this way, the effect of random biases may be reduced, and a single value may be used. As an alternative to averaging, a weighted average may be carried out, or any other suitable method of combining the risk predictions into a single risk value.

In some embodiments, the input cardiovascular signal may comprise at least one of: a single-lead ECG signal; an ambulatory monitoring signal; and a PPG signal. This allows for data from simple ECG devices, ambulatory monitors, or PPG sensors to be used.

In some embodiments, the input cardiovascular signal may comprise cardiovascular data for a window of at least one hour's length. Data from an hour window, either continuous or discontinuous, provides data beneficial for determining a risk of ventricular arrhythmias.

In some embodiments, the method may further comprise obtaining demographic data of the subject, and wherein the machine-learning model may comprise a first random forest classifier, the first random forest classifier being trained to predict, for the input cardiovascular signal and the demographic data, a demographic ventricular arrhythmia risk prediction. The use of a random forest classifier is effective in analyzing high-dimensional data, such as the cardiovascular signal and demographic data, e.g. age, sex, weight, etc. Demographic data can provide useful information for determining a ventricular arrhythmia risk prediction.

In some embodiments, prior to providing the at least one input cardiovascular signal as an input to the machine-learning model, the method may further comprise processing the input cardiovascular signal so that the input cardiovascular signal comprises at least one cardiovascular measurement value. Converting the cardiovascular signal into cardiovascular measurement values can provide the data in a more efficient and effective format for the machine-learning model to process. For example, the measurement values may comprise at least one of: premature ventricular contraction (PVC) burden; PVC QRS duration; non-sustained ventricular tachycardia (VT) frequency; PVC coupling interval; and heart rate variability (HRV).

In some embodiments, the machine-learning model may comprise a second random forest classifier, the second random forest classifier being trained to predict, for the at least one cardiovascular measurement value, a measurement ventricular arrhythmia risk prediction. It is beneficial for the converted cardiovascular measurement values to be input into a random forest classifier, which is effective in analyzing high-dimensional data, and so can predict a more reliable and accurate ventricular arrhythmia risk prediction based on the measurement values.

In some embodiments, prior to providing the at least one input cardiovascular signal as an input to the machine-learning model, the method may further comprise: processing the input cardiovascular signal so that the input cardiovascular signal comprises a heart rate density plot. Processing the cardiovascular signal to convert it into a heart rate density plot may provide data regarding the subject's heart rate in a more efficient and effective format.

In some embodiments, the machine-learning model may comprise a first neural network, the first neural network being trained to predict, for the heart rate density plot, a heart rate ventricular arrhythmia risk prediction. This allows a neural network, specifically trained to process heart rate density plots, to predict a ventricular arrhythmia risk while taking into account information about the subject's heart rate. For instance, the first neural network may comprise: a convolutional neural network trained to extract feature maps from the heart rate density plot; and an attention module configured to process the feature maps in order to predict a ventricular arrhythmia risk prediction. This may capture information about the subject's automatic nervous system and trigger.

In some embodiments, the machine-learning model may comprise a second neural network, the second neural network being trained to predict, for the input cardiovascular signal, a raw ventricular arrhythmia risk prediction. This allows a second neural network, specifically trained to process the raw input cardiovascular signal (or specifically, raw ECG or PPG signals), to predict a ventricular arrhythmia risk prediction taking into account information present in the raw input cardiovascular signal. For instance, the second neural network may comprise a convolutional neural network and an attention module. Thirty second cardiovascular strips may be extracted from the input cardiovascular signal every 5 minutes of, for example, a 24-hour long recording and passed to the convolutional neural network. This can then extract features from the strips. The extracted features can then be passed to the attention module which can then identify relationships of each beat from the strip to predict a cardiovascular-strip prediction for each strip respectively. All cardiovascular-strip predictions can then be averaged to generate the raw ventricular arrythmia risk prediction.

In some embodiments, determining a risk value may comprise comparing the ventricular arrythmia score to at least one threshold value and determining the risk value based on the comparison result. Comparing the risk score to a threshold value can facilitate a computationally efficient way to determine the risk value. For instance, if the risk score is over the threshold value, the risk value may comprise a high-risk label. If the risk score is under the threshold value, the risk value may comprise a low-risk label.

In some embodiments, the risk score may consist of a single risk prediction.

In some embodiments, the input cardiovascular signal may comprise an input ECG signal.

In some embodiments, the input cardiovascular signal may comprise an input PPG signal.

In some embodiments, the input cardiovascular signal may comprise cardiovascular data for a window of at least two hours, four hours, six hours, eight hours, ten hours, twelve hours, fourteen hours, sixteen hours, eighteen hours, twenty hours, twenty-two hours, or twenty-four hours length. Data from these windows of varying length, especially twenty-four hours, either continuous or discontinuous, can provide data beneficial for determining a risk of ventricular arrhythmia. The longer the window, the more accurate and/or reliable the predicted risk may be.

According to another aspect of the invention, there is provided a computer program comprising code means for implementing the method of any herein disclosed method when said program is run on a processing system.

According to another aspect of the invention, there is provided a system for predicting a risk of ventricular arrhythmia in a subject, the system comprising: an input interface configured to: obtain an input cardiovascular signal of the subject; and a processing unit configured to: provide the input cardiovascular signal as an input to a machine-learning model, the machine-learning model being trained to predict, based on the input cardiovascular signal, a ventricular arrhythmia risk score indicating a risk of future ventricular arrhythmia occurrence; and determine a risk value describing a predicted risk of future ventricular arrythmia occurrence for the subject based on the ventricular arrythmia score.

According to another aspect of the invention, there is provided a monitoring system comprising a cardiovascular monitor and the system disclosed above.

Thus, there may be proposed concepts for predicting a risk of ventricular arrhythmia in a subject, and this may be done based on the processing of an input cardiovascular signal.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for predicting a risk of ventricular arrhythmia in a subject according to a proposed embodiment;
Fig. 2A is a simplified flow diagram of a method for predicting a risk of ventricular arrhythmia in a subject involving cardiovascular measurement values according to a proposed embodiment;
Fig. 2B is a simplified flow diagram of a method for predicting a risk of ventricular arrhythmia in a subject involving cardiovascular measurement values according to a proposed embodiment;
Fig. 3 is a more in-depth flow diagram of a method for predicting a risk of ventricular arrhythmia in a subject according to a proposed embodiment;
Fig. 4 is a more in-depth flow diagram of a method for predicting a risk of ventricular arrhythmia in a subject using a random forest classifier according to a proposed embodiment;
Fig. 5 is a more in-depth flow diagram of a method for predicting a risk of ventricular arrhythmia in a subject involving a heart rate density plot according to a proposed embodiment;
Fig. 6 is a more in-depth flow diagram of a method for predicting a risk of ventricular arrhythmia in a subject according to a proposed embodiment;
Fig. 7 is a simplified block diagram of a system for predicting a risk of ventricular arrhythmia in a subject according to a proposed embodiment;
Fig. 8 is a simplified block diagram of a monitoring system comprising a cardiovascular monitor and the system of Fig. 7 according to a proposed embodiment; and
Fig. 9 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to predicting a risk of ventricular arrhythmia in a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for predicting a risk of ventricular arrhythmia in a subject. This can be achieved by utilizing a machine-learning model to process an input cardiovascular signal. The machine-learning model is trained to predict a ventricular arrhythmia risk score indicating a risk of future ventricular arrhythmia occurrence. The risk score can then be used to determine a risk value describing a predicted risk of future ventricular arrhythmia occurrence for the subject.

Proposed concepts thus aim to determine a risk value describing a predicted risk of future ventricular arrhythmia occurrence for the subject from a mere cardiovascular signal of a subject, e.g. an ECG or PPG signal. This can be done via the provision of the cardiovascular signal to a machine-learning model trained for this purpose. The predicted risk of future ventricular arrhythmia occurrence can be understood as the risk of ventricular arrhythmia occurring in the subject in the near-future, e.g. in the next month or two weeks or week or day, etc.

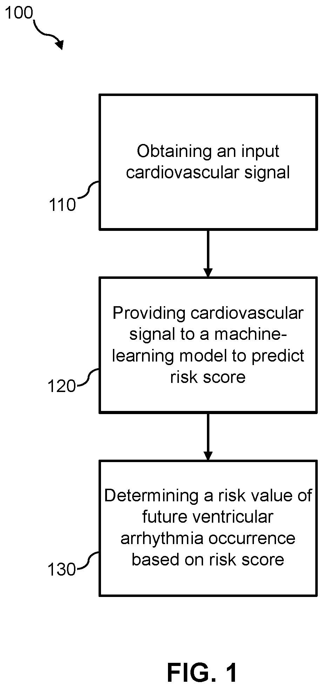

Referring now to Fig. 1, there is depicted a simplified flow diagram of a method 100 for predicting a risk of ventricular arrhythmia in a subject according to a proposed embodiment.

The method 100 begins with the step 110 of obtaining an input cardiovascular signal of the subject. The input cardiovascular signal may comprise at least one of: an ECG signal; and a PPG signal. More specifically, the input cardiovascular signal may comprise at least one of: a single-lead ECG signal; an ambulatory monitoring signal; and a PPG signal. This allows for data from simple ECG devices, ambulatory monitors, or PPG sensors to be used. A single-lead ECG recording can be collected using many different devices and remains agnostic of biases often found in clinical data. An ambulatory monitor, for example, may comprise a Holter monitor or Holter patch. An ambulatory monitoring signal may comprise at least one of: an ECG signal; and a PPG signal.

In this embodiment, the input cardiovascular signal comprises cardiovascular data for a window of at least one hour's length. Data from an hour window, either continuous or discontinuous, provides data beneficial for determining a risk of ventricular arrhythmia in the subject.

In other embodiments, however, the input cardiovascular signal may comprise cardiovascular data for a window of at least two hours, four hours, six hours, eight hours, ten hours, twelve hours, fourteen hours, sixteen hours, eighteen hours, twenty hours, twenty-two hours, or twenty-four hours length. Data from these windows of varying length, especially twenty-four hours, either continuous or discontinuous, can provide data beneficial for determining a risk of ventricular arrhythmia in the subject. The longer the window, the more accurate and/or reliable the predicted risk may be.

In step 120, the input cardiovascular signal is provided as an input to a machine-learning model, the machine-learning model being trained to predict, based on the input cardiovascular signal, a ventricular arrhythmia risk score indicating a risk of future ventricular arrhythmia occurrence. The machine-learning model may be developed and validated using a large database, for example, of 150,000 ambulatory ECG recordings. The machine-learning model may be a deep neural network that can be deployed in the cloud or embedded into a device.

In step 130, a risk value describing a predicted risk of future ventricular arrhythmia occurrence for the subject is determined based on the ventricular arrhythmia score. In this embodiment, a predicted risk of future ventricular arrhythmia occurrence may be understood as the risk of ventricular arrhythmia occurring in the subject in the next two-weeks. This future timeframe may vary, however. In other embodiments, the future timeframe may be the next week, the next forty-eight hours, the next twenty-four hours or even the next hour.

In this embodiment, the ventricular arrhythmia risk score is simply taken as the risk value. In other embodiments, however, the risk score may be adjusted in some way in order to determine the risk value. For example, the risk value may be a label, e.g. high-risk, medium-risk, or low-risk, wherein the risk score is compared to certain thresholds to determine the risk value. In other embodiments, the risk value may be a value between zero and one. In other embodiments, the risk value may be a percentage-likelihood of arrythmia occurrence in the near-future, this percentage being based off the risk score. In other embodiments, the risk value may be a set of values comprising a set of percentage-likelihoods of arrhythmia occurrences in a set of varying timeframes respectively. In yet other embodiments, the risk value may be an adjusted risk score, taking into account one or more further parameters, e.g. the risk score may be multiplied or divided by a certain adjustment value based on the subject's recent medical history, clinical diagnosis, or demographic data.

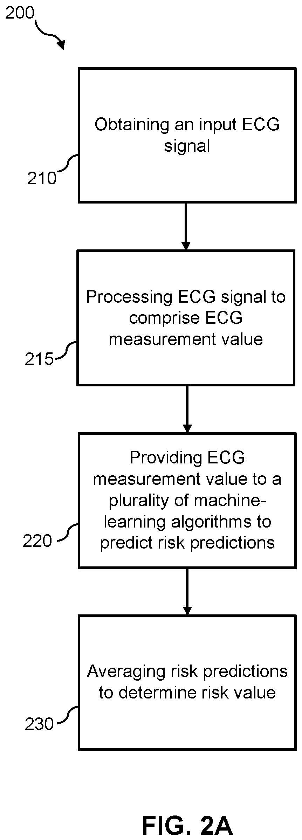

Referring now to Fig. 2A, there is depicted a simplified flow diagram of a method 200 for predicting a risk of ventricular arrhythmia in a subject involving cardiovascular measurement values according to a proposed embodiment.

The method 200 begins with step 210 of obtaining an input ECG signal. In step 215, the input ECG signal is processed so that the input ECG signal comprises at least one ECG measurement value. Converting the ECG signal into ECG measurement values can provide the data in a more efficient and effective format for the machine-learning model to process. For example, the measurement values may comprise at least one of: premature ventricular contraction (PVC) burden; PVC QRS duration; non-sustained ventricular tachycardia (VT) frequency; PVC coupling interval; and heart rate variability (HRV).

In step 220, the ECG measurement value(s) is provided to a plurality of machine-learning algorithms, each machine-learning algorithm generating a respective ventricular arrhythmia risk prediction. The use of different algorithms allows for more flexibility in the method with different factors being considered and processed differently. This can facilitate the provision of more efficient and/or reliable risk predictions that can ultimately result in a more efficient, effective and/or reliable risk score. In embodiments in which the machine-learning model comprises a plurality of machine-learning algorithms, each generating a respective ventricular arrhythmia risk prediction, the risk score thus comprises the plurality of risk predictions.

In step 230, the plurality of ventricular arrhythmia risk predictions are averaged to determine the risk value. In this way, the effect of random biases of each machine-learning algorithm may be reduced, and a single value may be used and output as the risk of future ventricular arrythmia occurrence in the subject. As an alternative to averaging, a weighted average may be carried out, or any other suitable method of combining the risk predictions into a single risk value.

In other embodiments, the method 200 of Fig. 2A may be performed with an PPG signal instead of an ECG signal, *mutatis mutandis.*

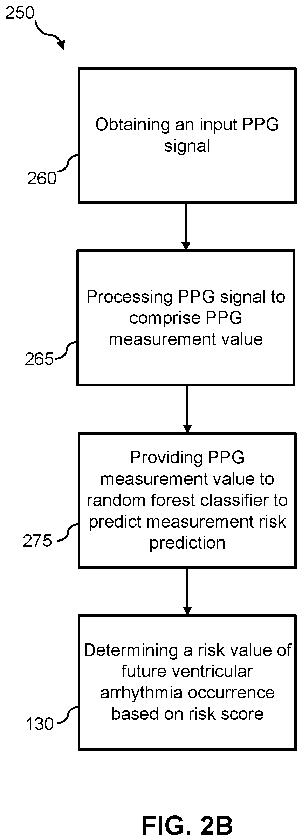

Referring now to Fig. 2B, there is depicted a simplified flow diagram of a method 250 for predicting a risk of ventricular arrhythmia in a subject involving PPG measurement values according to a proposed embodiment.

The method 250 begins with step 260 of obtaining an input PPG signal. Step 265 is much the same as step 215 described in relation to Fig. 2A above, however, with a PPG signal instead of an ECG signal.

In this embodiment, the machine-learning model comprises a random forest classifier trained to predict, for at least one PPG measurement value, a measurement ventricular arrhythmia risk prediction.

A random forest classifier is a machine learning algorithm that creates multiple decision trees and combines their outputs to make predictions. The algorithm randomly selects subsets of the data and features to build each decision tree, which helps to reduce overfitting and increase accuracy. When making a prediction, each tree in the forest produces a classification, and the final prediction is based on the majority vote of all trees. Random forest classifiers are effective for classification tasks in a variety of domains.

In step 275, the PPG measurement value(s) is thus provided to the random forest classifier in order to generate a measurement ventricular arrhythmia risk prediction. It is beneficial for the converted PPG measurement value(s) to be input into a random forest classifier, which is effective in analyzing high-dimensional data, and so can predict a more reliable and accurate ventricular arrhythmia risk prediction based on the measurement values as compared to other forms of machine-learning algorithms.

Thus, in this embodiment, the ventricular arrhythmia risk score consists of the single measurement ventricular arrhythmia risk prediction generated in step 275.

Step 130 is substantially the same as has been described above in relation to the method 100 of Fig. 1.

In other embodiments, the method 250 of Fig. 2B may be performed with an ECG signal instead of a PPG signal, *mutatis mutandis.*

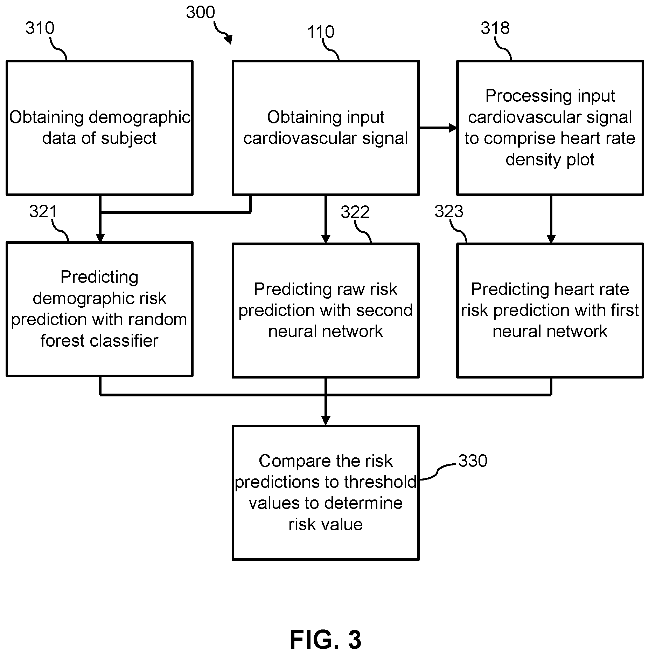

Referring now to Fig. 3, there is depicted a more in-depth flow diagram of a method 300 for predicting a risk of ventricular arrhythmia in a subject according to a proposed embodiment.

Step 110 is substantially the same as has been described above in relation to the method 100 of Fig. 1. In step 310, demographic data of the subject is obtained. Demographic data may comprise data describing at least one of the subject's age, sex, weight, ethnicity, etc. Demographic data can provide information that is useful for determining a ventricular arrhythmia risk prediction.

In step 321, the demographic data and the input cardiovascular signal are provided to a random forest classifier trained to predict, for the input cardiovascular signal and the demographic data, a demographic ventricular arrhythmia risk prediction. The use of a random forest classifier, as already stated, is effective in analysing high-dimensional data.

In step 318, the input cardiovascular input signal is processed so that the input cardiovascular signal comprises a heart rate density plot. Processing the cardiovascular signal to convert it into a heart rate density plot provides data regarding the subject's heart rate in a more efficient and effective format for subsequent processing.

In step 323, the heart rate density plot is provided to a first neural network, the first neural network being trained to predict, for the heart rate density plot, a heart rate ventricular arrhythmia risk prediction. This allows a neural network, specifically trained to process heart rate density plots, to predict a ventricular arrhythmia risk while taking into account information about the subject's heart rate.

Neural networks are a type of machine learning algorithm inspired by the structure and function of the human brain. They are composed of interconnected nodes (neurons) organized into layers, which process information and make predictions based on input data. The nodes in each layer receive input from nodes in the previous layer, perform a computation using weighted connections, and pass the result to the next layer. Neural networks can be trained using backpropagation, a method for adjusting the weights of the connections based on the difference between predicted and actual outputs.

In step 322, the input cardiovascular signal is provided to a second neural network, the second neural network being trained to predict, for the input cardiovascular signal, a raw ventricular arrhythmia risk prediction. This allows a second neural network, specifically trained to process the raw input cardiovascular signal (or specifically, raw ECG or PPG signals), to predict a ventricular arrhythmia risk while taking into account information present in the raw input cardiovascular signal.

In step 330, the three ventricular arrhythmia risk predictions generated in steps 321, 322, and 323 respectively are compared to three respective threshold values, and a risk value is determined based on the comparison results. Comparing the risk predictions to threshold values can facilitate a computationally efficient way of determining the risk value. For instance, if the risk predictions are over the threshold values, the risk value may comprise a high-risk label. If the risk predictions are under the threshold values, the risk value may comprise a low-risk label. If the risk predictions are a mixture of over and under the threshold values respectively, the risk value may comprise a medium-risk label. In other embodiments, step 330 may be replaced with a suitable alternative for determining a risk value based on the risk predictions, for instance, the risk predictions may simply be averaged to produce the risk value. In other embodiments, the risk value may comprise a weighted average of the risk predictions. In other embodiments, the risk predictions may be provided to a machine-learning algorithm trained to predict a risk value for the risk predictions.

The three main factors that cause ventricular arrythmias can be described as substrate, autonomic nervous system and trigger. Method 300 thus proposes an ensemble of three machine-learning algorithms, each considering one or more of these factors. For example, the first neural network predicting a heart rate risk prediction captures and utilizes information from the autonomic nervous system and trigger; the second neural network predicting a raw risk prediction captures and utilizes information from the arrhythmogenic substrate; and the random forest classifier predicting a demographic risk prediction captures and utilizes information from autonomic nervous system and triggers.

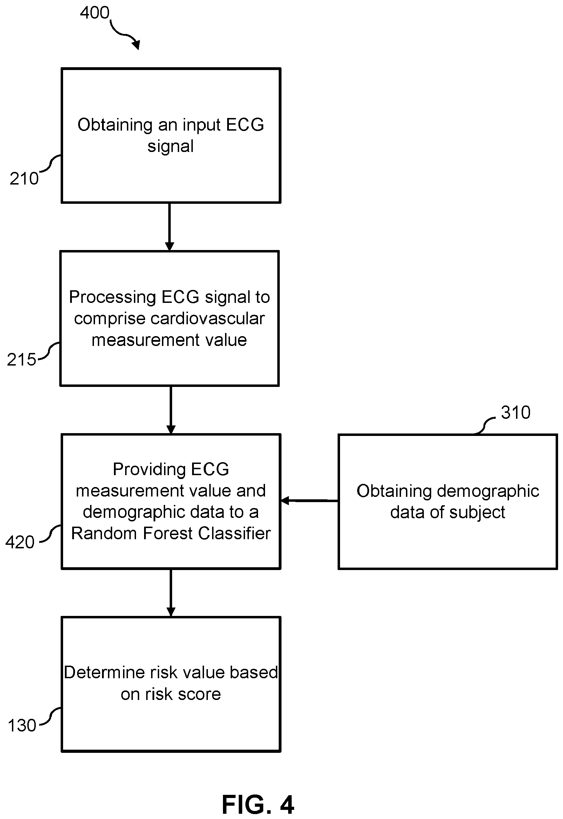

Referring now to Fig. 4, there is depicted a more in-depth flow diagram of a method 400 for predicting a risk of ventricular arrhythmia in a subject using a random forest classifier according to a proposed embodiment. Steps 210 and 215 are substantially the same as those described in relation to method 200 of Fig. 2A. Step 310 is substantially the same as that described in relation to method 300 of Fig. 3.

In this embodiment, the machine-learning model comprises a random forest classifier trained to predict, for at least one ECG measurement value and demographic data, a demographic ventricular arrhythmia risk prediction.

In step 420, the ECG measurement value(s) is thus provided to the random forest classifier together with the demographic data in order to generate a demographic ventricular arrhythmia risk prediction. It is beneficial for the converted ECG measurement value(s) along with the demographic data to be input into a random forest classifier, which is effective in analyzing high-dimensional data and so can predict a more reliable and accurate ventricular arrhythmia risk prediction based on the measurement values as compared to other forms of machine-learning algorithms.

Thus, in this embodiment, the ventricular arrhythmia risk score consists of the single demographic ventricular arrhythmia risk prediction generated in step 420.

Step 130 is substantially the same as that described in relation to method 100 of Fig. 1.

In other embodiments, method 400 may be performed with an input PPG signal instead of an input ECG signal, *mutatis mutandis.*

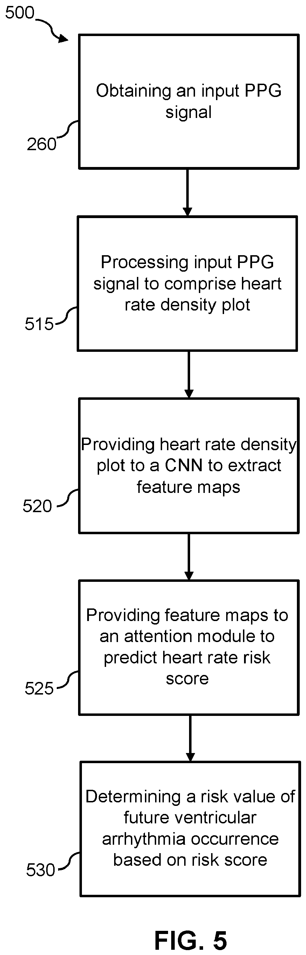

Referring now to Fig. 5, there is depicted a more in-depth flow diagram of a method 500 for predicting a risk of ventricular arrhythmia in a subject involving a heart rate density plot according to a proposed embodiment. Step 260 is substantially the same as that described in relation to Fig. 2B.

In step 515, the input PPG signal is processed so as to comprise a heart rate density plot. In step 520, the heart rate density plot is provided to a convolutional neural network (CNN) trained to extract feature maps from the heart rate density plot.

CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

In step 525, the extracted feature maps are provided to an attention module to predict a heart rate ventricular arrhythmia risk score. For example, the attention module may aggregate the temporal information across the extracted feature maps in order predict the risk score. In this embodiment, the attention module is a multi-head attention module, however, in other embodiments, it may be any other suitable attention module.

In step 530, a risk value is determined based on the ventricular arrhythmia risk score predicted in step 525. For example, the risk value may simply be taken as the risk score or it may be adjusted in some way.

In other embodiments, method 500 may be performed with an input ECG signal instead of an input PPG signal, *mutatis mutandis.*

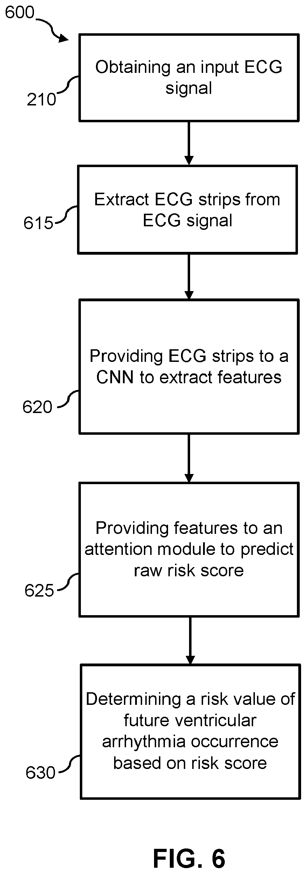

Referring now to Fig. 6, there is depicted a more in-depth flow diagram of a method 600 for predicting a risk of ventricular arrhythmia in a subject according to a proposed embodiment. Step 210 is substantially the same as that described in relation to method 200 of Fig. 2A. In this embodiment, the input ECG signal is a 24-hour long recording.

In step 615, thirty-second ECG strips are extracted from the input ECG signal every five minutes of the 24-hour long recording. In other embodiments, the strips may be extracted at any suitable frequency, such as, for example, every ten minutes, every fifteen minutes, every twenty minutes, every half an hour, etc. The input ECG signal may also be of any suitable length, for instance, from thirty minutes to 48 hours.

In step 620, the ECG strips are provided to a CNN configured to extract features from the strips. It should be noted that the CNN of step 620 may either be different to or the same as the CNN of step 520 in relation to method 500 of Fig. 5.

In step 625, the extracted features are provided to an attention module (which may be different or the same to the attention module of step 525 in relation to method 500 of Fig. 5) which then identifies relationships of each beat from each respective strip to predict an ECG-strip prediction for each strip respectively. All ECG-strip predictions are then averaged to predict a raw ventricular arrhythmia risk score. In this embodiment, the attention module is a multi-head attention module, however, in other embodiments, it may be any other suitable attention module.

In step 630, a ventricular arrythmia risk value is determined based on the risk score generated in step 625. For example, the risk value may simply be taken as the risk score or it may be adjusted in some way.

In some embodiments, method 600 may be performed with an input PPG signal instead of an input ECG signal, *mutatis mutandis.*

According to another aspect of the invention, there is provided a computer program comprising code means for implementing the method of any herein disclosed method when said program is run on a processing system.

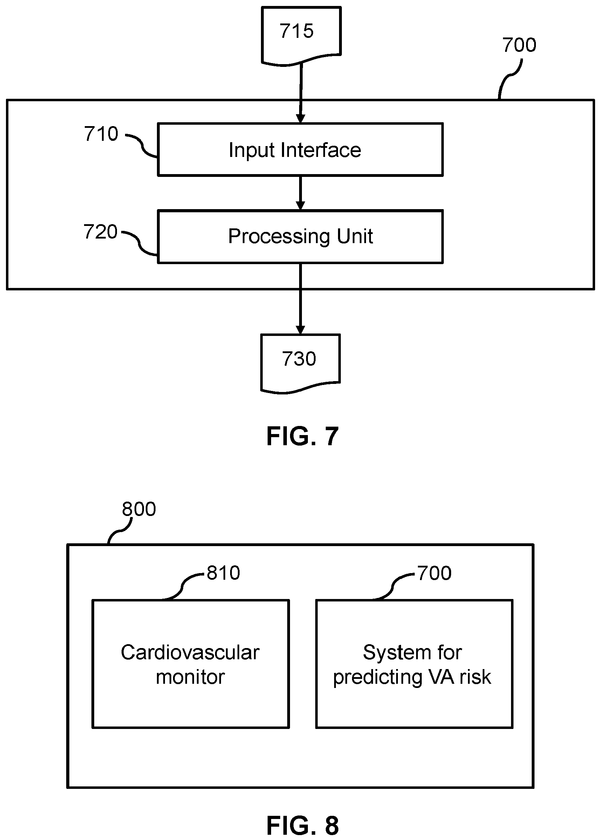

Referring now to Fig. 7, there is depicted a system 700 for predicting a risk of ventricular arrhythmia in a subject according to a proposed embodiment. The system 700 comprises an input interface 710 and a processor 720.

The system 700 is configured to predict a risk of ventricular arrhythmia in a subject by processing inputs 715. The inputs 715 comprise an input cardiovascular signal of the subject. The input interface 710 obtains the inputs 715 and the processor 720 is then configured to: provide the input cardiovascular signal as an input to a machine-learning model, the machine learning model being trained to predict, based on the input cardiovascular signal, a ventricular arrhythmia risk score indicating a risk of future ventricular arrhythmia occurrence; and based on this risk score, determine an output 730. The output 730 comprises a risk value describing a predicted risk of future ventricular arrythmia occurrence for the subject.

Referring now to Fig. 8, there is depicted a monitoring system 800 comprising a cardiovascular monitor 810 and the system 700 of Fig. 7. The cardiovascular monitor 810 may comprise at least one of: a single-lead ECG monitor; a Holter monitor; an ambulatory monitor; an ECG-sensing device; and a PPG-sensing device. An ambulatory monitor may obtain ECG and/or PPG signals. In some embodiments, the cardiovascular monitor 810 may be a smart watch or an ICU monitor.

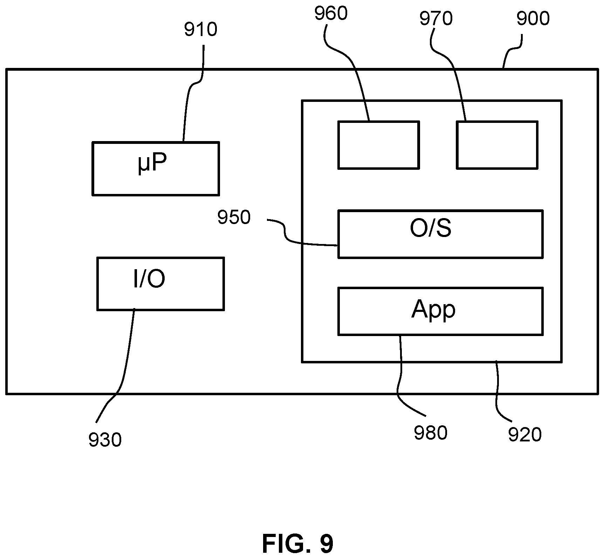

Fig. 9 illustrates an example of a computer 900 within which one or more parts of an embodiment may be employed. Various operations discussed above may utilize the capabilities of the computer 900. In this regard, it is to be understood that system functional blocks can run on a single computer or may be distributed over several computers and locations (e.g. connected via internet).

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor-based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 930 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 800 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method (100) for predicting a risk of ventricular arrhythmia in a subject, the method comprising:
obtaining an input cardiovascular signal (110) of the subject;
providing the input cardiovascular signal as an input to a machine-learning model (120), the machine-learning model being trained to predict, based on the input cardiovascular signal, a ventricular arrhythmia risk score indicating a risk of future ventricular arrhythmia occurrence; and
determining a risk value (130) describing a predicted risk of future ventricular arrhythmia occurrence for the subject based on the ventricular arrhythmia score.

2. The method of claim 1, wherein the machine-learning model comprises a plurality of machine-learning algorithms, wherein each machine-learning algorithm generates a respective ventricular arrhythmia risk prediction, and wherein the ventricular arrhythmia risk score comprises the plurality of ventricular arrhythmia risk predictions.

3. The method of claim 2, wherein determining the risk value based on the ventricular arrhythmia score comprises averaging the plurality of ventricular arrhythmia risk predictions (230).

4. The method of any prior claim, wherein the input cardiovascular signal comprises at least one of: a single-lead ECG signal; an ambulatory monitoring signal; and a PPG signal.

5. The method of any prior claim, wherein the input cardiovascular signal comprises cardiovascular data for a window of at least one hour's length.

6. The method of any prior claim, wherein the method further comprises obtaining demographic data of the subject (310), and wherein the machine-learning model comprises a first random forest classifier, the first random forest classifier being trained to predict, for the input cardiovascular signal and the demographic data, a demographic ventricular arrhythmia risk prediction.

7. The method of any prior claim, wherein prior to providing the at least one input cardiovascular signal as an input to the machine-learning model, the method further comprises:
processing the input cardiovascular signal (215) so that the input cardiovascular signal comprises at least one cardiovascular measurement value.

8. The method of claim 7, wherein the machine-learning model comprises a second random forest classifier, the second random forest classifier being trained to predict, for the at least one cardiovascular measurement value, a measurement ventricular arrhythmia risk prediction.

9. The method of any prior claim, wherein prior to providing the least one input cardiovascular signal as an input to the machine-learning model, the method further comprises:
processing the input cardiovascular signal (318) so that the input cardiovascular signal comprises a heart rate density plot.

10. The method of claim 9, wherein the machine-learning model comprises a first neural network, the first neural network being trained to predict, for the heart rate density plot, a heart rate ventricular arrhythmia risk prediction.

11. The method of any prior claim, wherein the machine-learning model comprises a second neural network, the second neural network being trained to predict, for the input cardiovascular signal, a raw ventricular arrythmia risk prediction.

12. The method of any prior claim, wherein determining a risk value comprises comparing the ventricular arrythmia score to at least one threshold value (330) and determining the risk value based on the comparison result.

13. A computer program comprising code means for implementing the method of any preceding claim when said program is run on a processing system.

14. A system (700) for predicting a risk of ventricular arrhythmia in a subject, the system comprising:
an input interface (710) configured to:
obtain an input cardiovascular signal of the subject; and
a processing unit (720) configured to:
provide the input cardiovascular signal as an input to a machine-learning model, the machine-learning model being trained to predict, based on the input cardiovascular signal, a ventricular arrhythmia risk score indicating a risk of future ventricular arrhythmia occurrence; and
determine a risk value describing a predicted risk of future ventricular arrhythmia occurrence for the subject based on the ventricular arrythmia score.

15. A monitoring system (800) comprising a cardiovascular monitor (810) and the system (700) of claim 14.
